# EUROPEAN PATENT APPLICATION

(11) **EP 2 172 224 A1**
(43) Date of publication of application: **07.04.2010**
(21) Application number: 08777618.3
(22) Date of filing: 26.06.2008
(51) Int. Cl.: A61K 47/38, A61K 9/08, A61K 45/00, A61K 47/10, A61K 47/12, A61K 47/34, A61K 47/36

(54) **AQUEOUS COMPOSITION**

(30) Priority: 26.06.2007 JP 2007167404
(71) Applicant: Wakamoto Pharmaceutical Co., Ltd., Chuo-ku Tokyo 103-8330 (JP)
(72) Inventor: SUZUKI, Hidekazu, Tokyo 103-8330 (JP)
(74) Representative: Vossius & Partner
(86) International application number: PCT/JP2008/061653
(87) International publication number: WO 2009/001899

(57) **Abstract**

The present invention provides an aqueous composition obtained by dissolving, in water, hydroxyethyl cellulose, methyl cellulose and/or Hypromelose and the invention also provides a medicament comprising the aqueous composition and a drug incorporated therein.

The viscosity of the composition of the present invention is abruptly increased when heated at a temperature near the body temperature, but it is rapidly reduced when applying weak force thereto, for instance, by lightly shaking the same. When the medicament obtained by incorporating a drug into the composition of the present invention is administered to a living body, the composition of the present invention can be thickened without delay at the administered site and thus stay at that site over a long period of time and this in turn leads to a considerable increase of the BA of the drug. In addition, the thermally thickened composition of the present invention can easily be converted into a composition having a high flow ability simply by applying weak force to the composition and therefore, the composition of the invention is not necessarily stored at a cold place or in a refrigerator and it is convenient to carry about.

## Description

### Technical Field

The present invention relates to an aqueous composition which can be thickened at a temperature near the human body temperature and in which the flow ability of the thickened composition is increased through the application of force.

### Background Art

Patent Document 1 specified below discloses reversibly thermally gelable aqueous pharmaceutical composition which comprises methyl cellulose and which can be gelatinized at the body temperature. This composition has such advantages that the administration thereof is easy because it is in a liquid state prior to its administration and that the composition permits the improvement of the ability of the drug included therein to stay at or remain in the administered site and likewise the improvement of the bioavailability (BA) thereof, since the liquid composition undergoes gelation at the body temperature after the administration thereof to thus increase its viscosity. At present, an eye drop has been put into practical use while making the most use of the characteristic properties of such a composition.
Patent Document 2 specified below discloses that a reversibly thermally gelable aqueous pharmaceutical composition permits the increase in the amount of the lacrimal fluid and the protection of the oily phase of the lacrimal fluid, as compared with the conventional artificial lacrimal fluid and that the aqueous pharmaceutical composition would considerably be effective for use as an artificial lacrimal fluid.
Patent Document 3 specified below discloses a composition which comprises methyl cellulose in combination with a substance capable of increasing the thixotropic properties thereof such as sugar alcohol, lactose or Carmelose (carboxymethyl cellulose). The composition may possibly cause gelation due to the increase of the atmospheric temperature during the summer season, but the composition can increase the flow ability of such a gelled composition and convert the gel into a sol simply by lightly shaking the gelled composition. This composition would be effective as an artificial lacrimal fluid quite handy to carry.
Patent Document 4 specified below discloses an ophthalmological composition free of any gelation, which comprises the following combination of two kinds of polymers: a combination of hydroxypropylmethyl cellulose and guar gum; hydroxypropylmethyl cellulose and a carboxyvinyl polymer; a carboxyvinyl polymer and guar gum; hydroxypropylmethyl cellulose and hydroxyethyl cellulose; hyaluronic acid and hydroxypropylmethyl cellulose; or hyaluronic acid and guar gum, wherein these combinations of two kinds of polymers each show a synergistic effect on the enhancement of the viscosities of the resulting compositions. This Patent Document likewise states that the composition can be used as an artificial lacrimal fluid and a vehicle for an ophthalmological drug.

In most of the eye drops, it is common that the eye drop is dropped in the eyes several times a day and accordingly, it is, in general, necessary for the user to carry about the eye drop for using the same. In addition, if the efficacy-sustaining term of the eye drop can be maintained over a longer time period, the number of drops thereof to be applied to the eyes is accordingly reduced and this may lighten the patient's burden. However, the eye drop may undergo significant changes in its physical properties through the gelation thereof and accordingly, it would be considered that the patient will feel uncomfortable with a foreign substance in his eyes. Moreover, there has not yet been known any composition which may gradually undergo its gelation and may instantaneously increase the viscosity thereof after the gelation thereof.

As has been described above, there has been known, as a technique for preparing a composition which never undergoes gelation upon the application thereof in the eyes, one in which the viscosity of the resulting composition is improved by the use of a combination of two kids of polymers in an amount of a low concentration relative to the total amount of the composition, but there has not yet been known any technique for the development of a composition whose viscosity is changed through the physical stimulation, for instance, by applying heat to the composition and/or by making the same vibrate.

Patent Document 1: Japanese Patent No. 2,729,859;
Patent Document 2: JP 2003-095924 A;
Patent Document 3: WO2005/042026;
Patent Document 4: JP 2007-500244 A.

### Disclosure of the Invention

### Problems to be Solved by the Invention:

It is a problem of the present invention to provide an aqueous composition in which it shows an abrupt increase in the viscosity thereof at a temperature near the body temperature, but the increased viscosity thereof is rapidly reduced upon the application of weak force, for instance, when lightly shaking the same to increase the flow ability thereof, i.e., an aqueous composition having an ability of being reversibly thermally thickened and having thixotropic properties (hereafter referred to as "reversibly thermally thickenable and thixotropic aqueous composition").

### Means for Solving the Problems

The present invention has been completed on the basis of such a finding that the foregoing problem can be solved by providing an aqueous solution which comprises hydroxyethyl cellulose and at least one member selected from the group consisting of methyl cellulose and Hypromelose (hydroxypropyl methyl cellulose). According to the present invention, there is thus provided an aqueous composition below:
1. An aqueous composition comprising an aqueous solution which contains (A) hydroxyethyl cellulose and (B) at least one member selected from the group consisting of methyl cellulose and Hypromelose.
2. The aqueous composition according to the foregoing item 1, wherein the concentration of the hydroxyethyl cellulose (A) ranges from 0.01 to 10% (w/v) and that of the at least one member selected from the group consisting of methyl cellulose and Hypromelose (B) ranges from 0.01 to 5% (w/v).
3. The aqueous composition according to the foregoing item 1 or 2, wherein the component (B) is methyl cellulose and wherein the composition further comprises (C) at least one member selected from the group consisting of sugar alcohols, polyvinyl pyrrolidone, citric acid or pharmaceutically acceptable salts thereof and hyaluronic acid or pharmaceutically acceptable salts thereof.
4. The aqueous composition according to the foregoing item 1 or 2, wherein the component (B) is Hypromelose and wherein the composition further comprises (C) at least one member selected from the group consisting of polyvinyl pyrrolidone, citric acid or pharmaceutically acceptable salts thereof and hyaluronic acid or pharmaceutically acceptable salts thereof.
5. The aqueous composition according to the foregoing item 4, wherein the composition further comprises (C) a sugar alcohol.
6. The aqueous composition according to any one of the foregoing items 1 to 5, wherein the composition further comprises (D) a drug.
7. The aqueous composition according to the foregoing item 6, wherein the drug (D) is at least one member selected from the group consisting of anti-infective agents, antiallergic agents, anti-inflammatory agents, glaucoma-treating agents, and corneal disorder-treating or dry eye-treating agents.
8. The aqueous composition according to any one of the foregoing items 1 to 7, wherein the composition is in the form of an injection, an orally administrable agent, an ear drop, a nasal drop, an eye drop or a liniment.

### Effects of the Invention

When the reversibly thermally thickenable and thixotropic aqueous composition according to the present invention is administered to a living body such as the human body, it can easily be thickened by the action of the body temperature thereof. Moreover, the composition of the present invention can undergo thickening even at a high environmental temperature experienced in, for instance, the summer season. However, the flow ability of the composition can be increased simply by lightly shaking the same because of the thixotropic properties thereof and accordingly, the composition can easily be administered to a living body.

It has in general been known that the viscosity of a high molecular weight compound-containing aqueous solution decreases as the temperature thereof is raised. In case of the composition of the present invention, however, when preparing shear stress-viscosity curves, for the composition, at the observation temperatures of 20°C and 35°C, the aqueous composition is found to have such characteristic properties that the viscosity thereof as determined at 35°C is higher than that determined at 20°C. Further, when the composition of the present invention once thickened by heating it to 35°C is cooled to 20°C, the viscosity of the composition is brought back to that prior to the application of heat and when the composition cooled down to 20°C is again heated to 35°C, it again undergoes thickening. In other words, the composition of the present invention shows a reversibly thermally thickening ability. In addition, the composition of the present invention has such characteristic properties that the viscosity of the composition according to the present invention thickened by heating it to 35°C is abruptly lowered by the application, to the thickened composition, of weak external force and the viscosity thus reduced may be brought back to its original level when the force applied thereto is removed. In other words, the composition of the present invention shows reversible thixotropic properties.

### Best Mode for Carrying Out the Invention

The viscosity of the component (A) or hydroxyethyl cellulose (hereafter abbreviated as "HEC") used in the present invention is not restricted to any specific range, but it is desirable to use, as such a component, those each having a viscosity preferably ranging from 3 to 10,000 mPa·s, as determined using a 2% (w/v) aqueous solution thereof at 20°C. Any HECs can be used alone or in any combination inasmuch as the viscosity thereof falls within the range specified above. The content of hydroxy-ethoxyl groups (the substitution rate of hydroxy groups present in the cellulose) preferably ranges from 30 to 70% from the viewpoint of the solubility thereof in water. Moreover, the HECs are distinguished one from others on the basis of their viscosities of the aqueous solution thereof. For instance, there have been known various kinds of commercially available ones having nominal viscosities of 10, 20, 300, 400 and 10,000 (each numeral means the viscosity value (mPa·s) as determined at 20° C using a 2% (w/v) aqueous solution thereof) and they are easily commercially available. It is preferred to use HECs having nominal viscosities preferably ranging from 10 to 400 because of their easy handleability. The outline, specification (standard), applications, amount to be used and trade names of HECs are detailed in "The Dictionary of Medicinal Additives" (edited by the Society of Medicinal Additives in Japan, published by YAKUJI-NIPPO Publishing Company).

The concentration of the HEC used in the composition of the present invention is not restricted to any specific range insofar as it may ensure the achievement of the effects of the present invention, but the concentration thereof preferably ranges from 0.01 to 10% (w/v), more preferably 0.05 to 4% (w/v), and most preferably 0.1 to 2% (w/v). The use of the HEC in a concentration of not more than 10% (w/v) is preferred, since the viscosity of the resulting composition falls within an easily handleable range, while the use of the HEC in a concentration of not less than 0.01% (w/v) is preferred, since the resulting composition can easily be thickened through heating.

The viscosity of methyl cellulose (hereafter abbreviated as "MC") used as the component (B) in the present invention is not restricted to any specific range, but MCs desirably used herein are those each having a viscosity as determined at 20°C using a 2% (w/v) aqueous solution thereof preferably ranging from 3 to 12,000 mPa·s. Any MCs can be used alone or in any combination inasmuch as the viscosity thereof falls within the range specified above. The content of methoxy groups (the substitution rate of hydroxyl groups present in the cellulose) preferably ranges from 26 to 33% from the viewpoint of the solubility in water. Moreover, the MCs are distinguished one from others on the basis of their viscosities of the aqueous solution. For instance, there have been known various kinds of commercially available ones having nominal viscosities of 4, 15, 25, 100, 400, 1,500 and 8,000 (each numeral means the viscosity value (mPa·s) as determined at 20°C using a 2% (w/v) aqueous solution thereof) and they are easily commercially available. It is preferred to use MCs each having nominal viscosity preferably ranging from 4 to 400 because of their easy handleability. The outline, specification (standard), applications, amount to be used and trade names of MCs are detailed in "The Dictionary of Medicinal Additives" (edited by the Society of Medicinal Additives in Japan, published by YAKUJI-NIPPO Publishing Company).

The concentration of the MC used in the composition of the present invention is not restricted to any specific range inasmuch as it may ensure the achievement of the effects of the present invention, but the concentration thereof preferably ranges from 0.01 to 5% (w/v), more preferably 0.05 to 3% (w/v), and most preferably 0.2 to 1.7% (w/v). The use of the MC in a concentration of not more than 5% (w/v) is preferred, since the viscosity of the resulting composition falls within an easily handleable range, while the use of the MC in a concentration of not less than 0.01% (w/v) is preferred, since the resulting composition can easily be thickened through heating.

The viscosity of Hypromelose (hereafter abbreviated as "HPMC") used as the component (B) in the present invention is not restricted to any specific range, but HPMCs desirably used herein are those each having a viscosity preferably ranging from 2 to 18,000 mPa·s, as determined at 20°C using a 2% (w/v) aqueous solution thereof. Any HPMCs can be used alone or in any combination inasmuch as the viscosity thereof falls within the range specified above. The content of methoxy groups (the substitution rate of hydroxy groups present in the cellulose) preferably ranges from 16 to 30% and the content of hydroxypropoxyl groups preferably ranges from 4 to 32%, from the viewpoint of the solubility in water. Moreover, the HPMCs are distinguished one from others on the basis of their viscosities of the aqueous solution. For instance, there have been known various kinds of commercially available ones having nominal viscosities of 3, 4, 4.5, 6, 15, 50, 100, 400, 4,000, 10,000 and 15,000 (each numeral means the viscosity value (mPa·s) as determined at 20°C using a 2% (w/v) aqueous solution thereof) and they are easily commercially available. It is preferred to use HPMCs each having nominal viscosity preferably ranging from 3 to 400 because of their easy handleability. The outline, specification (standard), applications, amount to be used and trade names of HPMCs are detailed in "The Dictionary of Medicinal Additives" (edited by the Society of Medicinal Additives in Japan, published by YAKUJI-NIPPO Publishing Company).

The concentration of the HPMC used in the composition of the present invention is not restricted to any specific range inasmuch as it may ensure the achievement of the effects of the present invention, but the concentration thereof preferably ranges from 0.01 to 5% (w/v), more preferably 0.05 to 2% (w/v), and most preferably 0.1 to 0.4% (w/v). The use of the HPMC in a concentration of not more than 5% (w/v) is preferred, since the viscosity of the resulting composition falls within an easily handleable range, while the use of the HPMC in a concentration of not less than 0.01% (w/v) is preferred, since the resulting composition can easily be thickened through heating.
When using, as the component (B), a combination of MC and HPMC in the composition of the present invention, the total concentration of the combination of MC and HPMC is not restricted to any specific range inasmuch as it may ensure the achievement of the effects of the present invention, but the concentration thereof preferably ranges from 0.01 to 7% (w/v), more preferably 0.05 to 5% (w/v), and most preferably 0.1 to 1.7% (w/v). The use of the combination of MC and HPMC in a total concentration of not more than 7% (w/v) is preferred, since the viscosity of the resulting composition falls within an easily handleable range, while the use of the combination of MC and HPMC in a total concentration of not less than 0.01% (w/v) is preferred, since the resulting composition can easily be thickened through heating.

In the composition of the present invention, the ratio of the concentration of the component (A) and that of the component (B) preferably ranges from 0.01 to 500, more preferably 0.03 to 300 and most preferably 0.3 to 20 as expressed in terms of the ratio: Component (A) [% (w/v)]/Component (B) [% (w/v)].
It is preferred to add, to the composition of the present invention, at least one member selected from the group consisting of sugar alcohols, polyvinyl pyrrolidone (hereafter abbreviated as "PVC"), citric acid or pharmaceutically acceptable salts thereof and hyaluronic acid or pharmaceutically acceptable salts thereof. The amount of these substances to be added to the composition is not restricted to any specific range inasmuch as it may ensure the achievement of the effects of the present invention, but the amount thereof to be added usually ranges from 0.01 to 10% (w/v), preferably 0.03 to 7% (w/v), and most preferably 0.05 to 4% (w/v). In this connection, however, the composition simply comprising hydroxyethyl cellulose, Hypromelose and a sugar alcohol hardly shows the desired characteristic properties of the present invention, among the foregoing combinations of the compounds.

Preferably used herein as the sugar alcohols include, for instance, mannitol, xylitol and sorbitol. Examples of pharmaceutically acceptable salts of citric acid and hyaluronic acid are sodium salts and potassium salts.
Therefore, an aqueous composition according to a preferred embodiment of the present invention comprises 0.01 to 10% (w/v) of HEC as the component (A), 0.01 to 5% (w/v) of MC and/or HPMC as the component (B), and 0.01 to 10% (w/v) at least one member selected from the group consisting of sugar alcohols, PVC, citric acid or pharmaceutically acceptable salts thereof and hyaluronic acid or pharmaceutically acceptable salts thereof as the component (C).

An aqueous composition according to a more preferred embodiment of the present invention comprises 0.05 to 4% (w/v) of HEC as the component (A), 0.05 to 3% (w/v) of MC and/or HPMC as the component (B), and 0.03 to 7% (w/v) at least one member selected from the group consisting of sugar alcohols, PVC, citric acid or pharmaceutically acceptable salts thereof and hyaluronic acid or pharmaceutically acceptable salts thereof as the component (C).

The aqueous composition of the present invention may further comprise a drug as the component (D). Examples of such drugs usable herein include anti-infectives, for instance, amphotericin B, fluconazole, miconazole nitrate, colistin sodium methane-sulfonate, carbenicillin sodium, gentamicin sulfate, erythromycin, azithromycin, tobramycin, kanamycin, ciprofloxacin hydrochloride, lomefloxacin hydrochloride, ofloxacin, levofloxacin, pazufloxacin tosilate, gatifloxacin, moxifloxacin hydrochloride, acyclovir, ganciclovir, cidofovir, sorbzin, trifluoro-thymidine and tetracyclines such as doxycycline; antiallergic agents such as acitazanolast, levocabastine hydrochloride, ketotifen fumarate, sodium salt of cromoglicic acid and tranilast; anti-inflammatory agents such as betamethasone phosphate, dexamethasone, hydrocortisone, sodium diclofenac, pranoprofen, indometacin, sodium bromfenac, meloxicam, lornoxicam, ciclosporin, and tacrolimus; glaucoma-treating agents such as timolol maleate, bunazosin hydrochloride, latanoprost, tafluprost, bimaprost, travoprost, nipradilol, carteolol hydrochloride, isopropyl unoprostone, dorzolamide hydrochloride, and brinzolamide; and corneal disorder-treating and dry eye-treating agents, for instance, aminoethyl-sulfonic acid, amino acids, sodium chondroitin sulfate, sodium hyaluronate, and tetracyclines such as doxycycline. The amount of these drugs to be incorporated into the composition of the present invention is not restricted to any specific range, inasmuch as the concentration thereof is such that it can ensure the achievement of the desired effects of the present invention. For instance, the amount thereof to be incorporated into the composition is on the order of 0.001 to 10% (w/v).

It is preferred that the pH value of the composition of the present invention is in general adjusted to a level ranging from 4 to 10 and, in particular, 5 to 8. The pH value of the composition of the present invention is adjusted through the use of a variety of currently used pH-adjusting agents. An acid can, for instance, be used for this purpose and specific examples thereof include ascorbic acid, hydrochloric acid, gluconic acid, acetic acid, lactic acid, phosphoric acid, sulfuric acid, citric acid, tartaric acid, and boric acid. A base can also be used for the control of the composition's pH value and specific examples thereof include potassium hydroxide, calcium hydroxide, sodium hydroxide, magnesium hydroxide, borax, monoethanolamine, diethanolamine, triethanolamine, trometamol, and meglumine. Also usable herein as such pH-adjusting agents include, for instance, amino acids such as glycine, histidine, and ε-aminocaproic acid.

When preparing the composition of the present invention, pharmaceutically acceptable additives such as an isotonicity, a solubilizing agent, a preservative and a stabilizer can if necessary be incorporated into the composition in an amount falling within the range which never impairs the effects of the present invention. Examples of such isotonicity include saccharides such as glucose; propylene glycol, glycerin, sodium chloride, and potassium chloride. Examples of such solubilizing agents are Polysorbate 80, and polyoxyethylene-hardened castor oil. Examples of the foregoing preservatives include invert soaps such as benzalkonium chloride, benzethonium chloride, and chlorhexidine gluconate; parabens such as methyl p-hydroxy-benzoate, propyl p-hydroxy-benzoate, and butyl p-hydroxy-benzoate; alcohols such as chloro-butanol, phenylethyl alcohol, and benzyl alcohol; and organic acids and salts thereof such as sodium dehydro-acetate, sorbic acid and potassium salt of sorbic acid. In addition, other additives usable herein include, for instance, thickening agents such as polyvinyl alcohol, propylene glycol, diethylene glycol or sodium polyacrylate; and stabilizers such as ethylenediamine-tetraacetic acid and pharmaceutically acceptable salts thereof, tocopherol and derivatives thereof and sodium sulfite.

Embodiments of the method for the preparation of the aqueous composition of the present invention will now be described below.
There is dispersed MC or HPMC in sterilized and purified water heated to a temperature of not less than 70°C and the resulting dispersion is then ice-cooled. After the temperature of the dispersion is brought back to room temperature, there are added to and dissolved in the dispersion, HEC, a sugar alcohol, PVP, citric acid, hyaluronic acid, a drug, additives and then these components are thoroughly admixed together. The pH value of the resulting mixture is controlled, and the total volume of the mixture is adjusted by the addition of sterilized and purified water to thus give an aqueous composition of the present invention. When using the resulting composition of the present invention as an eye drop, the composition thus prepared is first sterilized by the filtration thereof through a membrane filter and then packed in a container such as a plastic eye drop bottle.

The aqueous composition of the present invention can be used as, for instance, an artificial lacrimal fluid, a liquid for aiding the installation of a contact lense, an injectable solution, an orally administrable agent, an ear drop, a nasal drop, an eye drop or a liniment, while making the most use of the characteristic properties of the aqueous composition.

The aqueous composition of the present invention has such an advantage that the composition permits the improvement of the ability of the drug included therein to stay at or remain in the administered site and the improvement of the bioavailability (BA) thereof, since the composition is easily thickened by the action of the body temperature encountered when it is administered to a living body. Moreover, the viscosity of the composition according to the present invention is increased due to the action of the heat at a high environmental temperature experienced in, for instance, the summer season. However, the composition is not necessarily be stored in a cool place, for instance, in a refrigerator, the flow ability of the composition increases simply by lightly shaking the same and accordingly, the composition is handy to carry about and it is thus highly convenient.

### Examples

### [Test Example 1]

To 0.2 g of 65SH400 (Metholose (registered trade mark) available from Shin-Etsu Chemical Co., Ltd.), there was added 70 mL of sterilized and purified water heated to 85°C and then the mixture was stirred to give a dispersion. After confirming whether the material was uniformly dispersed in the water, the dispersion was ice-cooled with stirring. After ascertaining whether the dispersion became completely clear or not, it was allowed to stand till the temperature thereof was brought back to room temperature. To the resulting solution, there were added 0.2 g of sodium citrate, 4.0 g of mannitol, and 2.0 of polyvinyl pyrrolidone k25 (hereafter referred to as "PVPk25"), followed by the stirring of the mixture till these components were dissolved. Moreover, 1.5 g of HEC (available from Wako Pure Chemical Industries, Ltd.) was added to the resulting solution and the mixture was stirred till the HEC was completely dissolved. After ascertaining whether all of the components were dissolved or not, sterilized and purified water was added to the solution up to a total volume of 100 mL to thus give a composition of the present invention. The resulting composition was regarded as the sample of Example 1.

As Comparative Example, 2.0 g of HEC (available from Wako Pure Chemical Industries, Ltd.) was added to 80 mL of sterilized and purified water followed by the stirring of the resulting mixture till the former was completely dissolved in the latter. After confirming the complete dissolution of the HEC, sterilized and purified water was added to the solution up to a total volume of 100 mL to thus give a composition, which was regarded as the sample of Comparative Example 1.

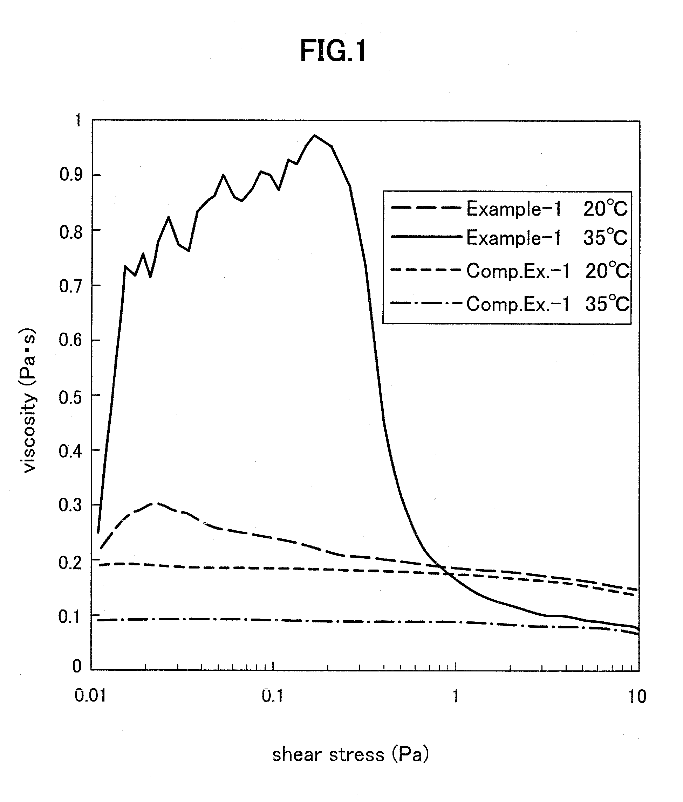

The compositions of Example 1 and Comparative Example 1 were evaluated or inspected for the relationship between the shear stress and the viscosity thereof observed at 20°C and 35°C.
The viscosity was determined using a viscometer AR2000 available from TA Instruments Company. About 2 mL of the composition of the present invention thus prepared was arranged between parallel plates of an acrylic resin having a diameter of 60 mm and a peltier for controlling the temperature. The gap between the parallel plates and the peltier was set at a level of 0.5 mm. After each sample for measurement was kept at that state at 20°C for 30 seconds, the temperature of the measuring system was raised up to a predetermined level (20°C or 35°C) and the sample was kept at that condition for additional 30 seconds. Then the viscosity of the sample was continuously determined while applying a shear stress ranging from 0.01 to 10 Pa to the sample. The results thus obtained are shown in Fig. 1 as a shear stress vs. viscosity curve.

As a result, it was found that the viscosity of the sample of Comparative Example, which used only HEC, was kept at a constant level, irrespective of the increase in the shear stress. This fact clearly indicates that the composition of Comparative Example is a Newtonian fluid. In addition, the viscosity thereof as determined at 35°C was, as a whole, lower than that observed at 20°C. This exactly corresponds to the behavior observed for the usual aqueous polymer solution.
In respect of the composition of the present invention, the viscosity thereof was lowered as the shear stress increased (at both 20°C and 35°C) and the composition of the present invention was thus proved to be a non-Newtonian fluid. This would indicate that some structures are formed within the aqueous composition.
Moreover, regarding the composition of Example 1, the comparison of the viscosity observed at 20°C with that observed at 35°C makes it clear that the latter is extremely high when the shear stress is not more than 1 Pa. This phenomenon is never observed for the usual polymeric aqueous composition like that observed for the sample of Comparative Example and the phenomenon is considered to be one which characterizes the ability of the composition according to the present invention to be reversibly thermally thickened. Accordingly, when incorporating a drug into the composition of the present invention which can rapidly be thickened at a temperature near the body temperature and administering the resulting product to a living body as a medicament, the composition of the present invention can rapidly be thickened at the administered site and remain or stay at that position over a long period of time and it would thus be expected that the BA of the drug can significantly be enhanced.
Moreover, the foregoing results also indicate that when the shear stress is increased to a level of not less than 1 Pa, the thickening effect due to the heat is abruptly attenuated and that the composition is converted into an aqueous composition having an extremely high flow ability. In particular, when the shear stress approaches about 10 Pa, the viscosity thereof as determined at 20°C becomes higher than that observed at 35°C. This phenomenon clearly indicates that the composition of the present invention thickened by the action of heat can easily be converted into one having a high flow ability by the application of weak force. In other words, even if the composition of the present invention is thickened during carrying about the same in, for instance, the summer season and the practical use thereof would seem to be difficult, the flow ability of the composition increases simply by lightly shaking the same and it can easily be administered to a living body.
Furthermore, it has been proved that the shear stress-viscosity curve as shown in Fig. 1 can be obtained even if the composition of the present invention is repeatedly heated and cooled, for instance, a sample thickened by heating to 35°C is cooled down to 20°C and then reheated to 35°C. This fact clearly indicates that the composition of the present invention has abilities to be reversibly, thermally thickened and to undergo thixotropic phenomenon.

### [Test Example 2]

To each of the predetermined amounts of SM-4, SM-100 and SN-400 (all of these substances are MCs, each available from Shin-Etsu Chemical Co., Ltd. under the trade name of Metholose™) and 65SH400, there was added 70 mL of sterilized and purified water heated to 85°C and then the mixture was stirred to give a dispersion. After confirming whether each component was uniformly dispersed in the water or not, the dispersion was ice-cooled with stirring. After ascertaining whether the dispersion became completely clear or not, it was allowed to stand till the temperature thereof was brought back to room temperature. To the resulting solution or composition, there was added each predetermined amount of sodium citrate, mannitol, sodium hyaluronate or PVPk25, depending on each particular composition, followed by the stirring of the mixture till each of these components was dissolved. Moreover, a predetermined amount of HEC (available from Wako Pure Chemical Industries, Ltd.) was added to the resulting solution and the mixture was stirred till the HEC was completely dissolved. After ascertaining whether all of the components were dissolved or not, sterilized and purified water was added to the solution up to a total volume of 100 mL. There were thus prepared compositions of Example 2 to Example 11.
The following Table 1 shows the formulations of Example 2 to Example 11 and Comparative Example 1, and the viscosity values (Pa·s) observed when a shear stress of 0.1 Pa or 10 Pa was applied to each composition which had been maintained at a temperature of 20°C or 35°C.

**[Table 1]**

| Component | | | Ex.2 | Ex.3 | Ex.4 | Ex.5 | Ex.6 | Ex.7 | Ex.8 | Ex.9 | Ex.10 |
|---|---|---|---|---|---|---|---|---|---|---|---|
| HEC w/v% | | | 2.0 | 2.0 | 2.0 | 2.0 | 2.0 | 2.0 | 2.0 | 2.0 | 1.5 |
| SM-4 w/v% | | | - | - | - | - | - | - | - | - | 0.3 |
| SM-100 w/v% | | | 0.5 | 0.5 | 0.5 | 0.5 | - | - | - | - | - |
| SM-400 w/v% | | | 0.1 | 0.1 | 0.1 | 0.1 | 0.2 | - | - | - | - |
| 65SH400 w/v% | | | - | - | - | - | - | 0.2 | 0.4 | 0.1 | 0.2 |
| PVPk25 w/v% | | | - | 3.0 | - | - | - | - | - | 0.5 | 2.0 |
| mannite w/v% | | | - | - | 4.0 | - | - | - | 1.0 | 1.0 | 0.2 |
| Na citrate w/v% | | | - | - | - | 3.0 | - | - | 2.0 | 2.0 | 0.4 |
| Na hyaluronate w/v% | | | - | - | - | - | 0.05 | 0.05 | - | - | - |
| viscosity (Pa·s) | shear stress 0.1Pa | 20°C | 0.502 | 0.767 | 0.178 | 0.605 | 0.718 | 0.330 | 1.133 | 0.613 | 0.213 |
| | | 35°C | 2.108 | 3.837 | 3.083 | 2.556 | 3.085 | 0.790 | 1.200 | 0.717 | 0.908 |
| | shear stress 10Pa | 20°C | 0.443 | 0.482 | 0.412 | 0.431 | 0.182 | 0.196 | 0.727 | 0.343 | 0.129 |
| | | 35°C | 0.254 | 0.289 | 0.244 | 0.241 | 0.104 | 0.109 | 0.366 | 0.175 | 0.069 |

The data listed in Table 1 indicate that regarding the samples of Examples 2 to 11, each of the viscosity values thereof as determined at 35°C are higher than each of the corresponding values as determined at 20°C, when the shear stress applied to them is 0.1 Pa, and that the samples undergo thickening by the action of heat. While if the shear stress applied to each samples is 10 Pa, the viscosity thereof as determined at 35°C is considerably low as compared with that observed when the shear stress applied to each samples is 0.1 Pa and the viscosity thereof as determined at 35°C is lower than that determined at 20°C. This fact clearly shows that even if the viscosity of the composition increases due to heat, the composition thus thickened can be converted into one having a high flow ability by the application of weak force thereto.
Moreover, the results summarized in Table 1 indicate that, in Examples 2 to 11, if a shear stress of 10Pa is applied to make the flow ability thereof high, then the composition is allowed to stand at 35°C for 30 seconds and thereafter, the shear stress is again reduced to 0.1 Pa, the viscosity of the composition is brought back to its initial level. This clearly proves that the composition of the present invention is reversible with respect to the force externally applied thereto, or that the composition of the present invention has abilities to be reversibly, thermally thickened and to undergo thixotropic phenomenon.

### [Test Example 3]

To 1.5 g of SM-4, there was added 70 mL of sterilized and purified water heated to 85°C and then the mixture was stirred to give a dispersion. After confirming whether the material was uniformly dispersed in the water or not, the dispersion was ice-cooled with stirring. After ascertaining whether the dispersion became completely clear or not, it was allowed to stand till the temperature thereof was brought back to room temperature. To the resulting solution, there were added 1.5 g of mannitol and 3.0 g of sodium citrate, followed by the stirring of the mixture till these components were dissolved. Moreover, 1.5 g of HEC (available from Wako Pure Chemical Industries, Ltd.) was added to the resulting solution and the mixture was stirred till the HEC was completely dissolved in the solution. After ascertaining whether all of the components were dissolved or not, a 1N NaOH solution or a 1N HCl solution was added to control the pH value of the solution to 7.4. Then sterilized and purified water was added to the solution up to a total volume of 100 mL to thus give a composition. The resulting composition was regarded as the sample of Example 12.

The product of the invention disclosed in International Patent Publication No: WO2005/ 042026 was herein regarded as the sample of Comparative Example 2 and the sample was prepared by repeating the same procedures used in the foregoing Example 12 except that HEC was not used.

The viscosity values of the compositions thus prepared were determined using a viscometer AR2000 available from TA Instruments Company. About 2mL of the composition of the present invention thus prepared was arranged between parallel plates of an acrylic resin having a diameter of 60 mm and a peltier for controlling the temperature. The gap between the parallel plates and the peltier was set at a level of 0.5 mm. After each sample for measurement was kept at that state at 20°C for 30 seconds, the temperature of the measuring system was raised up to 35°C and the sample was kept at that condition for additional 3 minutes. Then the viscosity of the sample was continuously determined while applying a shear stress ranging from 0.01 to 10 Pa to the sample.
The relation between the shear stress and the viscosity thus observed for the samples of Example 12 and Comparative Example 2 are summarized in the following Table 2.

**[Table 2]**

| Component | shear stress | Ex. 12 | Comp. Ex. 2 |
|---|---|---|---|
| HEC w/v% | | 1.5 | - |
| SM-4 w/v% | | 1.5 | 1.5 |
| mannitol w/v% | | 1.5 | 1.5 |
| Na citrate w/v% | | 3.0 | 3.0 |
| Viscosity at 35°C (Pa·s) | 0.5Pa | 34.79 | 20.17 |
| | 1.0Pa | 22.57 | 34.07 |
| | 1.5Pa | 0.455 | 38.70 |
| | 5.0Pa | 0.013 | 29.50 |
| | 10Pa | 0.005 | 0.005 |

The results listed in the foregoing Table 2 indicate that the viscosity of the sample of Example 12 is abruptly reduced when the shear stress is increased to a level of not less than 1.5 Pa and that the flow ability of the sample is increased. On the other hand, the data clearly indicate that the sample of Comparative Example 2 has a high viscosity even at a shear stress of 5.0 Pa and that the flow ability of the sample is not increased to a desired level unless stronger force is applied thereto.
This result shows that the viscosities of the both compositions increase in the same way upon heated to a temperature near the body temperature, but the composition of the present invention or the sample of Example 12 can be converted into a composition having a high flow ability by the application of force weaker than that required for increasing the flow ability of the sample of Comparative Example 2.

### [Test Example 4]

The viscosity was determined using a viscometer AR2000 available from TA Instruments Company. More specifically, the samples (2 mL each) of Example 12 and Comparative Example 2 prepared in Test Example 3 each were arranged between parallel plates of an acrylic resin having a diameter of 60 mm and a peltier for controlling the temperature. The gap between the parallel plates and the peltier was set at a level of 0.5 mm. Each sample for measurement was kept at that state at 20° C for 30 seconds. Thereafter, the temperature of the measuring system was raised up to 35°C over 25 seconds and the sample was then kept at a constant temperature of 35 °C. The viscosity of each sample was continuously determined immediately after the initiation of the temperature raise while applying a shear stress of 0.7 Pa to the sample.
The relation between the shear stress and the viscosity (shear stress: 0.7 Pa) thus observed for the samples of Example 12 and Comparative Example 2 are summarized in the following Table 3.

**[Table 3]**

| Component | | Example 12 | Comp. Ex. 2 |
|---|---|---|---|
| HEC w/v% | | 1.5 | - |
| SM-4 w/v% | | 1.5 | 1.5 |
| mannitol w/v% | | 1.5 | 1.5 |
| Na citrate w/v% | | 3.0 | 3.0 |
| Viscosity (Pa·s) | 0.25min | 0.245 | 0.003 |
| | 0.5min | 0.433 | 0.003 |
| | 1.0min | 1.053 | 0.004 |
| | 1.5min | 2.648 | 0.005 |

The data listed in Table 3 indicate that the viscosity of the sample of Example 12 increases immediately after the raising of the temperature and the viscosity thereof as determined at 1.5 minutes after raising the temperature is increased even to 10 times that observed after 0.25 minutes from the initiation of the temperature raise. On the other hand, the data listed in Table 3 indicate that the viscosity of the sample of Comparative Example 2 shows almost no change after the temperature raise and that the sample does not show any increase in its viscosity even if it is heated for a short period of time. The foregoing clearly indicates that the sample of Example 12 shows immediate response to the temperature raise or to heat, or that the sample sensitively responds to heat to thus cause an increase of its viscosity, while the sample of Comparative Example 2 never shows such sharp response to heat.

### [Test Example 5]

To 0.3 g of 65SH400, there was added 70 mL of sterilized and purified water heated to 85°C and then the mixture was stirred to give a dispersion. After confirming whether the material was uniformly dispersed in the water or not, the dispersion was ice-cooled with stirring. After ascertaining whether the dispersion became completely clear or not, it was allowed to stand till the temperature thereof was brought back to room temperature. To the resulting solution, there were added 4.0 g of mannitol and/or 0.5 or 3.0 g of PVPk25, and 3.5 g of sodium citrate, followed by the stirring of the mixture till these components were dissolved. Moreover, 0.1 g of HEC (available from Wako Pure Chemical Industries, Ltd.) was added to the resulting solution and the mixture was stirred till the HEC was completely dissolved in the solution. After ascertaining whether all of these components were dissolved or not, a 1N NaOH solution or a 1N HCl solution was added to control the pH value of the solution to 7.0. Then sterilized and purified water was added to the solution up to a total volume of 100 mL to thus give a composition. The resulting compositions were regarded as the samples of Examples 13 to 16.

The product of the invention disclosed in TOKUHYO 2007-500244 was herein regarded as the sample of Comparative Example and the sample thereof was prepared according to the following procedures.
To 0.3 g of 65SH400, there was added 70 mL of sterilized and purified water heated to 85°C and then the mixture was stirred to give a dispersion. After confirming whether the material was uniformly dispersed in water or not, the dispersion was ice-cooled with stirring. After ascertaining whether the dispersion became completely clear or not, it was allowed to stand till the temperature thereof was brought back to room temperature. To the resulting solution, there were added 4.0 g of mannitol, followed by the stirring of the mixture till the component was dissolved. Moreover, 0.1 g of HEC (available from Wako Pure Chemical Industries, Ltd.) was added to the resulting solution and the mixture was stirred till the HEC was completely dissolved in the solution. After ascertaining whether all of the foregoing components were dissolved or not, a 1N NaOH solution or a 1N HCl solution was added to adjust the pH value of the solution to 7.0. Then sterilized and purified water was added to the solution up to a total volume of 100 mL to thus give a composition. The resulting composition was regarded as the sample of Comparative Example 3.

The viscosity values of the compositions thus prepared were determined using a viscometer AR2000 available from TA Instruments Company. About 2 mL of the composition of the present invention thus prepared was arranged between parallel plates of an acrylic resin having a diameter of 60 mm and a peltier for controlling the temperature. The gap between the parallel plates and the peltier was set at a level of 0.5 mm. After each sample for measurement was kept at that state at 20°C for 30 seconds, the temperature of the measuring system was raised up to 20°C or 35°C and the sample was kept at that condition for additional 30 seconds. Then the viscosity of the sample was continuously determined while applying a shear stress ranging from 0.01 to 10 Pa to the sample. The relation between the shear stress and the viscosity thus observed for the samples of Examples 13 to 16 and Comparative Example 3 are summarized in the following Table 4.

**[Table 4]**

| Component | | Shear stress | Ex. 13 | Ex. 14 | Ex. 15 | Ex. 16 | Comp. Ex. 3 |
|---|---|---|---|---|---|---|---|
| HEC w/v% | | | 0.1 | 0.1 | 0.1 | 0.1 | 0.1 |
| 65SH400 w/v% | | | 0.3 | 0.3 | 0.3 | 0.3 | 0.3 |
| mannitol w/v% | | | 4.0 | 4.0 | - | - | 4.0 |
| PVPk25 w/v% | | | 0.5 | - | 3.0 | - | - |
| Na citrate w/v% | | | - | 3.5 | - | 3.5 | - |
| Viscosity (Pa·s) | 20°C | 0.05Pa | 0.007 | 0.007 | 0.011 | 0.023 | 0.006 |
| | | 0.1Pa | 0.007 | 0.007 | 0.009 | 0.009 | 0.006 |
| | | 10Pa | 0.007 | 0.007 | 0.007 | 0.006 | 0.006 |
| | 35°C | 0.05Pa | 0.012 | 0.320 | 0.408 | 1.156 | 0.004 |
| | | 0.1Pa | 0.009 | 0.008 | 0.421 | 1.129 | 0.004 |
| | | 10Pa | 0.004 | 0.005 | 0.005 | 0.004 | 0.004 |

Regarding the sample of Comparative Example 3, there is not observed any change of viscosity on and after the temperature raise, the viscosity of the composition as determined at 20°C is higher than that determined at 35°C, under the both shear stress conditions used and there is not observed any effect of heat on the thickening of the composition.
On the other hand, the samples of Examples 13 to 16 certainly show increases in viscosity on and after the temperature raise, the viscosity of each sample as determined at 35°C is higher than that determined at 20°C, at a shear stress of 0.05 Pa or 0.1 Pa and this indicates that each composition undergoes thickening at a temperature near the body temperature. The viscosity of each of the samples obtained in Examples 13 to 16 as determined at 35°C is lower than that determined at 20°C at a shear stress of 10 Pa. Thus it is clear that the composition of the present invention thickened by the application of heat can easily be converted into one having a high flow ability by the application of weak force thereto.
More specifically, the composition of Comparative Example 3 never shows any immediate response to heat and any thermal reversibility, while the compositions of Examples 13 to 16 certainly show the both immediate response to heat and thermal reversibility, or these compositions have abilities to be reversibly, thermally thickened and to undergo thixotropic phenomenon.

### [Test Example 6]

To a mixture of 0.5 g of SM-100 and 0.1 g of SM-400, there was added 70 mL of sterilized and purified water heated to 85°C and then the mixture was stirred to give a dispersion. After confirming whether these materials were uniformly dispersed in the water or not, the dispersion was ice-cooled with stirring. After ascertaining whether the dispersion became completely clear or not, it was allowed to stand till the temperature thereof was brought back to room temperature. To the resulting solution, there were added 2.0 g each of sodium citrate, mannitol and PVPk25, and desired amounts of a variety of drugs (levofloxacin, timolol maleate, acitazanolast, sodium diclofenac, betamethasone sodium phosphate, aqueous solution of azithromycin, and doxycycline hydrochloride), followed by the stirring of the mixture till these components were dissolved. Moreover, 1.5 g of HEC (available from Wako Pure Chemical Industries, Ltd.) was added to the resulting solution and the mixture was stirred till the HEC was completely dissolved in the solution. After ascertaining whether all of these components were dissolved or not, sterilized and purified water was added to the solution up to a total volume of 100 mL to thus give each of the corresponding samples of Examples 17 to 24. In this connection, the foregoing aqueous solution of azithromycin used herein was one prepared by dissolving 2.0 g of azithromycin and 0.4 g of citric acid monohydrate in sterilized and purified water and further adding sterilized and purified water to the resulting solution to make the total volume thereof 100 mL. The following Table 5 shows the formulations of Examples 17 to 24 and the viscosity values observed when a shear stress of 0.1 Pa or 10 Pa was applied to each sample which had been maintained at a temperature of 20°C or 35°C.

**[Table 5]**

| Component | | | Ex.17 | Ex.18 | Ex.19 | Ex.20 | Ex.21 | Ex.22 | Ex.23 | Ex.24 |
|---|---|---|---|---|---|---|---|---|---|---|
| HEC w/v% | | | 1.5 | 1.5 | 1.5 | 1.5 | 1.5 | 1.5 | 1.5 | 1.5 |
| SM-100 w/v% | | | 0.5 | 0.5 | 0.5 | 0.5 | 0.5 | 0.5 | 0.5 | 0.5 |
| SM-400 w/v% | | | 0.1 | 0.1 | 0.1 | 0.1 | 0.1 | 0.1 | 0.1 | 0.1 |
| levofloxacin w/v% | | | 0.5 | - | - | - | - | - | - | - |
| timolol maleate, w/v% | | | - | 0.68 | - | - | - | - | - | - |
| acitazanolast w/v% | | | - | - | 0.1 | - | - | - | - | - |
| Na diclofenac w/v% | | | - | - | - | 0.1 | - | - | - | - |
| azithromycin w/v% | | | - | - | - | - | 0.2 | - | - | - |
| citric acid 1H₂O w/v% | | | - | - | - | - | 0.04 | - | - | - |
| Na betamethasone phosphate, w/v% | | | - | - | - | - | - | 0.01 | - | - |
| dorzolamide hydrochlorid e w/v% | | | - | - | - | - | - | - | 1.0 | - |
| doxycycline hydrochloride w/v% | | | - | - | - | - | - | - | - | 0.02 |
| PVPk25 w/v% | | | 2.0 | 2.0 | 2.0 | 2.0 | 2.0 | 2.0 | 2.0 | 2.0 |
| mannite w/v% | | | 2.0 | 2.0 | 2.0 | 2.0 | 2.0 | 2.0 | 2.0 | 2.0 |
| Na citrate w/v% | | | 2.0 | 2.0 | 2.0 | 2.0 | 2.0 | 2.0 | 2.0 | 2.0 |
| Viscosity (Pa·s) | shear stress 0.1Pa | 20°C | 0.836 | 0.651 | 0.626 | 0.558 | 0.684 | 0.592 | 0.623 | 0.758 |
| | | 35°C | 4.321 | 2.581 | 3.055 | 3.353 | 2.113 | 1.787 | 2.035 | 3.321 |
| | shear stress 10Pa | 20°C | 0.424 | 0.410 | 0.414 | 0.414 | 0.407 | 0.447 | 0.442 | 0.425 |
| | | 35°C | 0.228 | 0.212 | 0.247 | 0.247 | 0.232 | 0.226 | 0.226 | 0.198 |

The data listed in Table 5 prove that the viscosity of each of the samples prepared in Examples 17 to 24 as determined at 35°C is higher than that determined at 20°C when the shear stress is set at 0.1 Pa and that the sample thus causes the thickening by the action of heat. In addition, when the shear stress is set at 10 Pa, the viscosity of each sample as determined at 35°C is extremely low as compared with that observed at a shear stress of 0.1 Pa and the viscosity thereof at 35°C is lower than that determined at 20°C. This clearly proves that the composition of the present invention containing a drug may be thickened due to heat, but the thickened composition can be converted into a composition having a high flow ability by the application of weak force.

### [Test Example 7]

To 0.2 g of SM-400, there was added 70 mL of sterilized and purified water heated to 85°C and then the mixture was stirred to give a dispersion. After confirming whether the material was uniformly dispersed in the water or not, the dispersion was ice-cooled with stirring. After ascertaining whether the dispersion became completely clear or not, it was allowed to stand till the temperature thereof was brought back to room temperature. To the resulting solution, there were added 1.0 g of sodium citrate, 1.0 g of xylitol or sorbitol, 2.0 g of PVPk25, 0.7 g of boric acid, 0.02 g of borax and a preservative (10 µ L of a 20% chlorhexidine gluconate solution or 20 µ L of a 50% benzalkonium chloride solution diluted 10 times with sterilized and purified water), followed by the stirring of the mixture till these components were dissolved. Moreover, 1.5 g of HEC (available from Wako Pure Chemical Industries, Ltd.) was added to the resulting solution and the mixture was stirred till the HEC was completely dissolved therein. After ascertaining whether all of these components were dissolved or not, sterilized and purified water was added to the solution up to a total volume of 100 mL to thus give each corresponding sample or composition. The resulting compositions were regarded as the samples of Examples 25 and 26.
The following Table 6 shows the formulations of Examples 25 and 26 and the viscosity values observed when a shear stress of 0.1 Pa or 10 Pa was applied to each sample which had been maintained at a temperature of 20°C or 35°C.

**[Table 6]**

| Component | | | Example 25 | Example 26 |
|---|---|---|---|---|
| SM-400 w/v% | | | 0.2 | 0.2 |
| HEC w/v% | | | 1.5 | 1.5 |
| sodium citrate w/v% | | | 1.0 | 1.0 |
| PVP k 25 w/v% | | | 2.0 | 2.0 |
| boric acid w/v% | | | 0.7 | 0.7 |
| borax w/v% | | | 0.02 | 0.02 |
| xylitol w/v% | | | 1.0 | - |
| sorbitol w/v% | | | - | 1.0 |
| chlorhexidine gluconate w/v% | | | 0.002 | - |
| benzalkonium chloride w/v% | | | - | 0.001 |
| Viscosity (Pa·s) | shear stress 0.1Pa | 20°C | 0.248 | 0.152 |
| | | 35°C | 0.659 | 0.308 |
| | shear stress 10Pa | 20°C | 0.121 | 0.104 |
| | | 35°C | 0.064 | 0.056 |

The data listed in Table 6 prove that the viscosity of each of the samples of Examples 25 and 26 as determined at 35°C is higher than that determined at 20°C when the shear stress is set at 0.1 Pa and that the sample accordingly causes the thickening by the action of heat. In addition, when the shear stress is set at 10 Pa, the viscosity of each sample as determined at 35°C is extremely low as compared with that observed at a shear stress of 0.1 Pa, the viscosity thereof at 35°C is extremely low and it is lower than that determined at 20°C. This clearly proves that the composition of the present invention may be thickened by the action of heat, but the thickened composition can be converted into a composition having a high flow ability by the application of weak force.

### Example 27 (Injection)

To a mixture of 5.0 g of SM-100 and 1 g of SM-400, there was added 70 mL of sterilized and purified water heated to 85°C and then the mixture was stirred to give a dispersion. After confirming whether the materials were uniformly dispersed in the water or not, the dispersion was ice-cooled with stirring. After ascertaining whether the dispersion became completely clear or not, it was allowed to stand till the temperature thereof was brought back to room temperature. To the resulting solution, there were added predetermined amounts of additives, i.e., 20 g of sodium citrate, 40 g of mannitol and 1 g of betamethasone sodium phosphate, followed by the stirring of the mixture till these components were dissolved. Moreover, 15 g of HEC was added to the resulting solution and the mixture was stirred till the HEC was completely dissolved therein. After ascertaining whether all of these components were dissolved or not, sterilized and purified water was added to the solution up to a total volume of one liter to thus give a composition of the present invention.
The resulting composition was filtered through a membrane filter, followed by dispensing and packaging the same into glass ampoules (each having a 5 mL volume) and melt-sealing them to give injections.

### Example 28 (Nasal Drop)

To a mixture of 5.0 g of SM-100 and 1 g of SM-400, there was added 70 mL of sterilized and purified water heated to 85°C and then the mixture was stirred to give a dispersion. After confirming whether the materials were uniformly dispersed in the water or not, the dispersion was ice-cooled with stirring. After ascertaining whether the dispersion became completely clear or not, it was allowed to stand till the temperature thereof was brought back to room temperature. To the resulting solution, there were added 20 g each of sodium citrate, mannitol and PVPk25 as well as 5 g of levofloxacin, followed by the stirring of the mixture till these components were dissolved. Moreover, 15 g of HEC (available from Wako Pure Chemical Industries, Ltd.) was added to the resulting solution and the mixture was stirred till the HEC was completely dissolved therein. Then a 1N NaOH solution was added to the solution to control the pH value thereof to 7.4 and sterilized and purified water was added to the solution up to a total volume of one liter to thus give a composition of the present invention. The resulting levofloxacin- containing composition of the present invention was filtered through a membrane filter, followed by packaging the same into plastic containers for dropping it in the nose and they were used as nasal drops.

### Example 29 (Ear Drop)

The azithromycin-containing composition of the present invention, prepared in Test Example 6 (Example 16, detailed in Table 5) was filtered through a membrane filter, followed by packaging the same into plastic containers and they were used as ear drops.

### Example 30 (Liniment)

The levofloxacin-containing composition of the present invention prepared in Example 28 was filtered through a membrane filter, followed by packaging the same into plastic containers and they were used as liniments.

### Example 31 (Orally Administrable Drug)

The azithromycin-containing composition of the present invention, prepared in Test Example 6 (Example 16, detailed in Table 5) was filtered through a membrane filter, followed by packaging the same into glass containers and they were used as orally administrable drugs.

### Example 32 (Eve Drop)

To 2 g of SM-400, there was added 700 mL of sterilized and purified water heated to 85°C and then the mixture was stirred to give a dispersion. After confirming whether the material was uniformly dispersed in the water or not, the dispersion was ice-cooled with stirring. After ascertaining whether the dispersion became completely clear or not, it was allowed to stand till the temperature thereof was brought back to room temperature. To the resulting solution, there were added 10 g of sodium citrate, 10 g of mannitol, 20 g of PVPk25, 7 g of boric acid, 0.2 g of borax, 6.8 g of timolol maleate and a preservative (0.1 mL of a 20% chlorhexidine gluconate solution), followed by the stirring of the mixture till these components were dissolved. Moreover, 15 g of HEC (available from Wako Pure Chemical Industries, Ltd.) was added to the resulting solution and the mixture was stirred till the HEC was completely dissolved therein. After ascertaining whether all of these components were dissolved or not, a 1N NaOH solution was added to the solution to control the pH value thereof to 7.4 and sterilized and purified water was added to the solution up to a total volume of one liter to thus give a timolol maleate-containing composition of the present invention. The resulting composition was filtered through a membrane filter, followed by packaging the same into 5 mL volume each of plastic containers for dropping it in the eyes and they were used as eye drops.

### Example 33 (Eye Drop)

To 2 g of SM-400, there was added 700 mL of sterilized and purified water heated to 85°C and then the mixture was stirred to give a dispersion. After confirming whether the material was uniformly dispersed in water or not, the dispersion was ice-cooled with stirring. After ascertaining whether the dispersion became completely clear or not, it was allowed to stand till the temperature thereof was brought back to room temperature. To the resulting solution, there were added 10 g of sodium citrate, 10 g of mannitol, 5 g of sodium hyaluronate, 7 g of boric acid, 0.2 g of borax and a preservative (0.1 mL of a 20% chlorhexidine gluconate solution), followed by the stirring of the mixture till these components were dissolved. Moreover, 15 g of HEC (available from Wako Pure Chemical Industries, Ltd.) was added to the resulting solution and the mixture was stirred till the HEC was completely dissolved therein. After ascertaining whether all of these components were dissolved or not, a 1N NaOH solution was added to the solution to control the pH value thereof to 7.4 and sterilized and purified water was added to the solution up to a total volume of one liter to thus give a composition of the present invention. The resulting composition was filtered through a membrane filter, followed by packaging the same into 5 mL volume each of plastic containers for dropping it in the eyes and they were used as eye drops.

### Example 34 (Artificial Lacrimal Fluid)

The composition of the present invention, prepared in Test Example 7 (Example 26, detailed in Table 6) was filtered through a membrane filter, followed by packaging the same into 5 mL volume each of plastic containers for eye drops and they were used as artificial lacrimal fluid.

### Industrial Applicability

When a medicament obtained by incorporating a drug into the composition of the present invention, which can abruptly be thickened by heating at a temperature near the body temperature is administered to a living body, the composition of the present invention can immediately be thickened at the administered site and thus stay at that site over a long period of time and this in turn enhances the BA of the drug, markedly. In addition, the thermally thickened composition of the present invention can easily be converted into a composition having a high flow ability simply by applying weak force to the composition and therefore, the composition of the invention is not necessarily stored at a cold place or in a refrigerator and is convenient to carry about.

### Brief Description of the Drawings

Fig. 1 is a graph showing the relation between the shear stress and the viscosities observed for the compositions prepared in Example 1 and comparative Example 1 as determined at temperatures of 20 and 35°C.

## Claims

1. An aqueous composition comprising an aqueous solution which contains (A) hydroxyethyl cellulose and (B) at least one member selected from the group consisting of methyl cellulose and Hypromelose.

2. The aqueous composition according to claim 1, wherein the concentration of the hydroxyethyl cellulose (A) ranges from 0.01 to 10% (w/v) and that of the at least one member selected from the group consisting of methyl cellulose and Hypromelose (B) ranges from 0.01 to 7% (w/v).

3. The aqueous composition according to claim 1 or 2, wherein the component (B) is methyl cellulose and wherein the composition further comprises (C) at least one member selected from the group consisting of sugar alcohols, polyvinyl pyrrolidone, citric acid or pharmaceutically acceptable salts thereof and hyaluronic acid or pharmaceutically acceptable salts thereof.

4. The aqueous composition according to claim 1 or 2, wherein the component (B) is Hypromelose and wherein the composition further comprises (C) at least one member selected from the group consisting of polyvinyl pyrrolidone, citric acid or pharmaceutically acceptable salts thereof and hyaluronic acid or pharmaceutically acceptable salts thereof.

5. The aqueous composition according to claim 4, wherein the composition further comprises (C) a sugar alcohol.

6. The aqueous composition according to any one of claims 1 to 5, wherein the composition further comprises (D) a drug.

7. The aqueous composition according to claim 6, wherein the drug (D) is at least one member selected from the group consisting of anti-infective agents, antiallergic agents, anti-inflammatory agents, glaucoma-treating agents, and corneal disorder-treating or dry eye-treating agents.

8. The aqueous composition according to any one of claims 1 to 7, wherein the composition is in the form of an injection, an orally administrable agent, an ear drop, a nasal drop, an eye drop or a liniment.
